# EUROPEAN PATENT APPLICATION

(11) **EP 3 549 519 A1**
(43) Date of publication of application: **09.10.2019**
(21) Application number: 18166123.2
(22) Date of filing: 06.04.2018
(51) Int. Cl.: A61B 5/024, A61B 5/11, A61B 5/20, A61B 5/01

(54) **METHOD AND APPARATUS FOR MONITORING A SUBJECT**

(71) Applicant: Koninklijke Philips N.V., 5656 AE Eindhoven (NL)
(72) Inventor: PFUNDTNER, Stefan, 5656 AE Eindhoven (NL); BULUT, Murtaza, 5656 AE Eindhoven (NL); AARTS, Ronaldus Maria, 5656 AE Eindhoven (NL)
(74) Representative: de Haan, Poul Erik

(57) **Abstract**

According to an aspect, there is provided a method of monitoring a subject, the method comprising obtaining, from a sensor, a signal indicative of the movements of the subject over time; obtaining, from a sensor, a signal indicative of the heart rate of the subject over time; and processing the obtained signal indicative of the movements and the obtained signal indicative of the heart rate to identify a urination event of the subject as a time period where a level of movement by the subject is below a threshold and the heart rate decreases.

## Description

### FIELD OF THE INVENTION

The invention relates to a method and apparatus for monitoring a subject, and in particular a method and apparatus for monitoring a subject to identify a urination event of the subject.

### BACKGROUND OF THE INVENTION

Urination (or micturition, or voiding, or uresis) is characterized by the released of urine from the bladder through the urethra outside the body. In healthy subject, the process of urination is under voluntary control, and occurs in average five (5) to eight (8) times a day. Increased urination frequency and/or duration, among other things, within a subject may be indicative of health disorders.

The monitoring of the urination pattern of a subject such as frequency and duration can be used, among other things, to assess the health status, disease development and the treatment progression of people. For example there is a need to monitor people suffering from urinary-related diseases and prostate diseases, and people receiving diuretics (so-called water pills). Frequent urination can be a sign of a health issue, like diabetes. Disturbed urination patterns can be sign of stress and anxiety. The progress of other diseases or conditions can also be observed from urination patterns. For example, an overactive bladder (OAB) is characterised by frequent urination events, and lower urinary tract symptoms can be caused by benign prostate hyperplasia which is characterised by difficulties in starting the flow of urine and the complete emptying of the bladder. An overactive bladder is estimated to occur in 7-27% of men and 9-43% of women, and becomes more common with aging.

One of the monitoring tool for OAB is to keep a micturition diary, which is not practical to do by hand, and is subject to mistakes. Alternatively, one of the therapies of AOB is to procrastinate the next time of micturition, with a timer set after the previous micturition, a threshold might be given that the subject needs to wait some time to visit the toilet the next time.

Several methods and techniques have been proposed for urination monitoring, such as keeping a urination (or micturition) diary, but these methods and techniques do not provide reliable, automatic, objective or continuous detection and monitoring of urination events.

A technique has been proposed in WO 2017/162465 that detects urination events based on changes of skin temperature of a subject over time. However, it has been found that some subjects do not exhibit a detectable skin temperature change.

There is therefore a need for an improved means to support the monitoring and/or detecting of urination events of a subject.

### SUMMARY OF THE INVENTION

It is an object of the invention to provide for an improved method and apparatus for non-intrusively monitoring a subject to identify urination events of a subject in a reliable and objective manner.

According to a first specific aspect, the object is realized by a method of monitoring a subject, the method comprising obtaining, from a sensor, a signal indicative of the movements of the subject over time; obtaining, from a sensor, a signal indicative of the heart rate of the subject over time; and processing the obtained signal indicative of the movements and the obtained signal indicative of the heart rate to identify a urination event of the subject as a time period where a level of movement by the subject is below a threshold and the heart rate decreases. Thus this method provides an alternative approach to detecting urination events from measurements of the subject that can be obtained automatically and unobtrusively, while providing reliable and objective information or data.

In some embodiments, the step of processing comprises identifying a urination event of the subject as a time period where a level of movement by the subject is below a threshold and the heart rate decreases to close to a resting heart rate of the subject, to the resting heart rate of the subject, or below the resting heart rate of the subject.

In some embodiments, the method further comprises one of (i) processing the signal indicative of the heart rate of the subject over time to determine the resting heart rate of the subject; and (ii) receiving a measurement of the resting heart rate of the subject.

In some embodiments, the step of processing the obtained signal indicative of the movements further comprises processing the obtained signal indicative of the movements to detect periods of time in which the subject is walking; and identifying the urination event as a time period where the level of movement by the subject is below a threshold and the heart rate decreases, and where the time period is preceded and followed by a period of time in which the subject is detected to be walking. These embodiments improve the reliability of the urination event detection as in almost all cases there will be periods of walking before and after each urination event.

In some embodiments, the step of processing the obtained signal indicative of the movements further comprises processing the obtained signal indicative of the movements to detect the posture of the subject over time; and identifying the urination event as a time period where the level of movement by the subject is below a threshold and the heart rate decreases, and where the subject is in a sitting or standing posture. These embodiments further improve the reliability of the urination event detection as the subject is likely to be in a sitting or standing posture during the urination event.

In some embodiments, the step of processing further comprises processing a PPG measurement signal from a PPG sensor to determine a signal representing the modulation depth of the PPG measurement signal over time; and identifying a urination event of the subject as a time period where the level of movement by the subject is below the threshold and (i) the heart rate decreases; and/or (ii) the modulation depth decreases. These embodiments further improve the reliability of the urination event detection as a correlation has been found between the occurrence of urination events and decreases in the modulation depth.

In some embodiments, the method further comprises the step of obtaining, from a sensor, a signal indicative of the skin temperature of the subject over time; and the step of processing comprises processing the obtained signal indicative of the skin temperature to identify time periods in which the skin temperature decreases; and identifying a urination event of the subject as a time period where a level of movement by the subject is below the threshold and (i) the heart rate decreases; and/or (ii) the skin temperature decreases. These embodiments further improve the reliability of the urination event detection as a correlation has been found between the occurrence of urination events and decreases in the skin temperature.

In some embodiments, the method further comprises the step of analysing one or more identified urination events to determine one or more characteristics of the urination event or events, the one or more characteristics comprising one or more of the duration of the urination event; the time of the urination event; the frequency with which urination events occur; an indication of pain, discomfort, stress, anxiety, emotional fluctuations and/or difficulty for the subject before, during and/or after the urination event. These embodiments can provide useful information for enabling or assisting a healthcare professional to assess the health of the subject.

In some embodiments, the signal indicative of the movements of the subject is obtained using a photoplethysmogram, PPG, sensor, or a movement sensor. These embodiments have the advantage that a single sensor is required to measure both the movements and heart rate of the subject.

In some embodiments, the signal indicative of the heart rate of the subject are obtained using a photoplethysmogram, PPG, sensor, an electrocardiogram, ECG, sensor or a movement sensor.

According to a second aspect, the object is realized by a computer program product comprising a computer readable medium having computer readable code embodied therein, the computer readable code being configured such that, on execution by a suitable computer or processor, the computer or processor is caused to perform the method according to the first aspect of any embodiment thereof.

According to a third aspect, the object is realized by an apparatus for monitoring a subject, the apparatus comprising a processing unit configured to obtain a signal indicative of the movements of the subject over time; obtain a signal indicative of the heart rate of the subject over time; and process the obtained signal indicative of the movements and the obtained signal indicative of the heart rate to identify a urination event of the subject as a time period where a level of movement by the subject is below a threshold and the heart rate decreases. Thus this apparatus provides an alternative approach to detecting urination events from measurements of the subject that can be obtained automatically and unobtrusively.

In some embodiments, the processing unit is configured to identify a urination event of the subject as a time period where a level of movement by the subject is below a threshold and the heart rate decreases to close to a resting heart rate of the subject, to the resting heart rate of the subject, or below the resting heart rate of the subject.

In some embodiments, the processing unit is further configured to (i) process the signal indicative of the heart rate of the subject over time to determine the resting heart rate of the subject; or (ii) obtain a measurement of the resting heart rate of the subject.

In some embodiments, the processing unit is configured to process the obtained signal indicative of the movements to detect periods of time in which the subject is walking; and identify the urination event as a time period where the level of movement by the subject is below a threshold and the heart rate decreases, and where the time period is preceded and followed by a period of time in which the subject is detected to be walking. These embodiments improve the reliability of the urination event detection as in almost all cases there will be periods of walking before and after each urination event.

In some embodiments, the processing unit is configured to process the obtained signal indicative of the movements to detect the posture of the subject over time; and identify the urination event as a time period where the level of movement by the subject is below a threshold and the heart rate decreases, and where the subject is in a sitting or standing posture. These embodiments further improve the reliability of the urination event detection as the subject is likely to be in a sitting or standing posture during the urination event.

In some embodiments, the processing unit is further configured to process a PPG measurement signal from a PPG sensor to determine a signal representing the modulation depth of the PPG measurement signal over time; and identify a urination event of the subject as a time period where the level of movement by the subject is below the threshold and (i) the heart rate decreases; and/or (ii) the modulation depth decreases. These embodiments further improve the reliability of the urination event detection as a correlation has been found between the occurrence of urination events and decreases in the modulation depth.

In some embodiments, the processing unit is further configured to obtain, from a sensor, a signal indicative of the skin temperature of the subject over time; wherein the processing unit is configured to process the obtained signal indicative of the skin temperature to identify time periods in which the skin temperature decreases; and identify a urination event of the subject as a time period where a level of movement by the subject is below the threshold and (i) the heart rate decreases; and/or (ii) the skin temperature decreases. These embodiments further improve the reliability of the urination event detection as a correlation has been found between the occurrence of urination events and decreases in the skin temperature.

In some embodiments, the processing unit is further configured to analyse one or more identified urination events to determine one or more characteristics of the urination event or events, the one or more characteristics comprising one or more of the duration of the urination event; the time of the urination event; the frequency with which urination events occur; an indication of pain, discomfort, stress, anxiety, emotional fluctuations and/or difficulty for the subject before, during and/or after the urination event. These embodiments can provide useful information for enabling or assisting a healthcare professional to assess the health of the subject.

In some embodiments, the processing unit is configured to obtain the signal indicative of the movements of the subject from a photoplethysmogram, PPG, sensor, or a movement sensor. In some embodiments, the apparatus further comprises the PPG sensor or the movement sensor. These embodiments have the advantage that a single sensor is required to measure both the movements and heart rate of the subject.

In some embodiments, the processing unit is configured to obtain the signal indicative of the heart rate of the subject from a photoplethysmogram, PPG, sensor, an electrocardiogram, ECG, sensor or a movement sensor. In some embodiments, the apparatus further comprises the PPG sensor, the ECG sensor or the movement sensor.

According to a fourth aspect, there is provided a system for monitoring a subject, the system comprising an apparatus according to the third aspect or any embodiment thereof, and one or more sensors for measuring the heart rate of the subject and the movements of the subject.

These and other aspects will be apparent from and elucidated with reference to the embodiment(s) described hereinafter.

It will be appreciated by those skilled in the art that two or more of the above-mentioned options, implementations, and/or aspects of the invention may be combined in any way deemed useful.

### BRIEF DESCRIPTION OF THE DRAWINGS

Exemplary embodiments will now be described, by way of example only, with reference to the following drawings, in which:
Fig. 1 comprises graphs showing skin temperature measurements for three subjects around respective urination events;
Fig. 2 comprises graphs showing heart rate measurements for three subjects around respective urination events;
Fig. 3 is a block diagram of an apparatus according to an embodiment of the invention;
Fig. 4 is a flow chart illustrating a method according to an embodiment of the invention;
Fig. 5 is a graph illustrating an exemplary measurement signal from an accelerometer;
Fig. 6 is a graph illustrating an exemplary measurement signal from a photoplethysmogram (PPG) sensor;
Fig. 7 comprises graphs showing changes in modulation depth of a PPG signal for three subjects around respective urination events; and
Fig. 8 is a flow chart illustrating another exemplary method of detecting urination events.

### DETAILED DESCRIPTION OF THE PREFERRED EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

As noted above, the monitoring of the urination pattern of a subject can be used to assess the health state and the treatment of the subject. Information on the urination patterns or urination events of a subject, including any one or more of the frequency (i.e. how often), the duration, whether there was difficulty starting urination, whether there was urgency to urinate, stop and go patterns during urination, whether the subject had to strain to urinate, pain and/or anxiety before, during or after urination, whether the subject had a feeling of incomplete emptying of the bladder and whether the subject needs to return to urinate shortly after finishing the previous event (e.g. a few minutes), are useful to a doctor or other healthcare professional for diagnosing certain medical conditions and/or tracking the progress and/or treatment of certain medical conditions. Therefore it would be useful to have an apparatus that can monitor a subject and identify urination events by the subject.

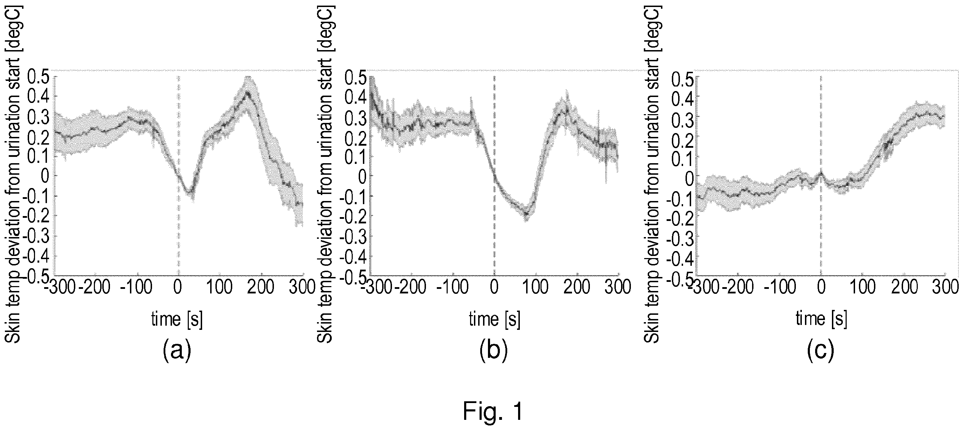

WO 2017/162465 describes that urination events can be detected based on changes of skin temperature of a subject, and in particular a drop in skin temperature that occurs during the urination event. Fig. 1 comprises three graphs that each show skin temperature measurements (and in particular a signal indicative of skin temperature) for three different subjects over a time period that included a urination event by each subject. The graphs show: a 40 year old healthy male (Fig. 1(a)), a 35 year old healthy male (Fig. 1(b)), and a 60 year old healthy male (Fig. 1(c)). The time axes of each graph have been aligned so that the urination (micturition) event for each subject occurs at time = 0, and the skin temperature in each graph is presented with reference to the skin temperature of the subject at the start of the urination event (i.e. at time = 0). It can be seen that there is a characteristic drop or fall in skin temperature for the first two subjects (Figs. 1(a) and 1(b)), but the skin temperature did not drop for the third subject (Fig. 1(c)).

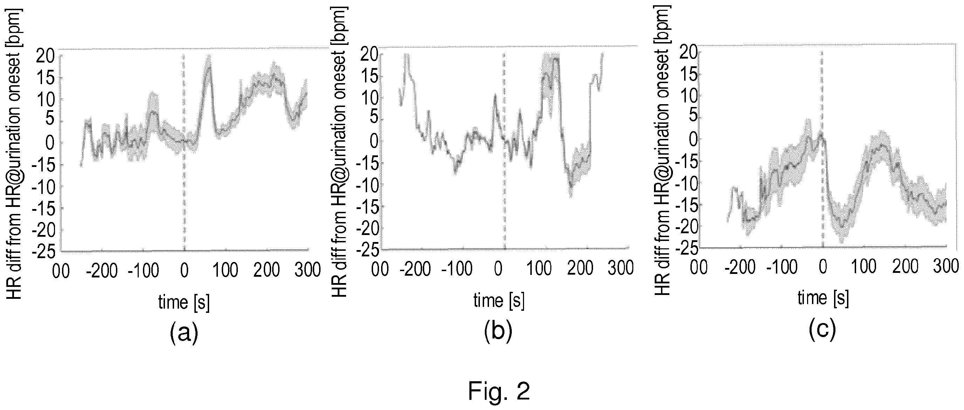

However, from further monitoring of these subjects it has been found that the third subject (shown in Fig. 1(c)) had a drop in their heart rate at the start of the urination event (time = 0). Fig. 2 comprises three graphs that each show heart rate measurements (and in particular a signal indicative of heart rate) for the same three subjects over the time period that included a urination event by each subject. As in Fig. 1, the time axes of each graph have been aligned so that the urination (micturition) event for each subject occurs at time = 0, and the heart rate measurements in each graph is presented with reference to the heart rate of the subject at the start of the urination event (i.e. at time = 0). It can be seen that there is no significant changes in the heart rate for the first two subjects (Figs. 2(a) and 2(b)) but there is a sudden drop or fall in heart rate for the third subject (Fig. 2(c)), with an increase in the heart rate following or later in the urination event.

Thus, it can be seen that monitoring the heart rate and observing a drop in heart rate could be used to detect a urination (micturition) event. The absence of a skin temperature drop and a brief episode of low (or lower) heart rate) may indicate that the subject is straining to urinate, which could in the future develop into a more problematic pathology like micturition syncope or lower urinary tract symptoms (LUTS). Thus, the invention proposes to detect a urination event based on evaluating a signal indicative of the heart rate of a subject. It will be appreciated that the heart rate of a subject can change for a variety of reasons, and so observing changes in heart rate alone would not provide a sufficiently high reliability rate, and therefore the invention also provides that a signal indicative of the movements of the subject is analysed to identify periods of time that might correspond to urination events (i.e. periods of time when the subject is relatively still and motionless, at least in terms of substantial motion such as walking, etc.), and then the heart rate is evaluated for these periods of time to determine if there has been a urination event. In some embodiments, as described in more detail below, a urination event can be detected by monitoring heart rate and skin temperature.

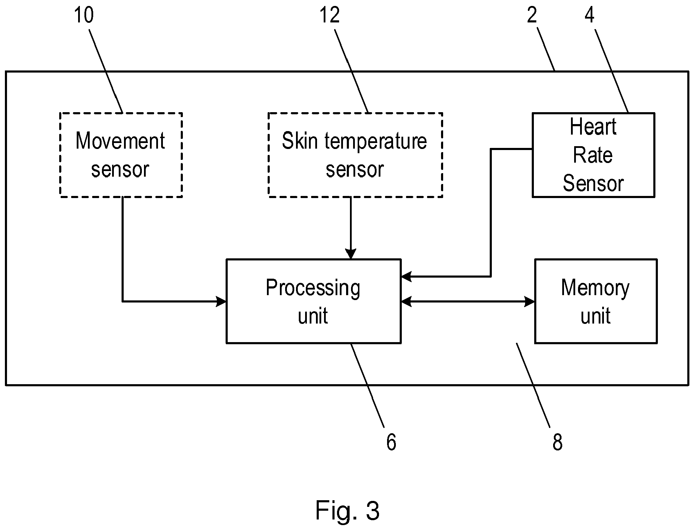

An embodiment of an apparatus 2 for monitoring a subject according to the invention is shown in Fig. 3. The apparatus 2 is preferably in a form that can be worn or carried by the subject in a generally unobtrusive manner. For example, the apparatus 2 can be in the form of a watch, wrist band, chest band, or any other type of device that can worn on or around a body part of the subject. In some embodiments the apparatus 2 can be integrated into an item of clothing such as a shirt or jumper. In some embodiments the apparatus 2 can be in a form that is suitable for wearing in or on the skin, for example a plaster or temporary tattoo.

The apparatus 2 comprises a heart rate sensor 4 that is for measuring the heart rate of the subject, or for providing measurements of the heart rate of the subject. The heart rate sensor 4 outputs a heart rate signal indicative of the heart rate of the subject over time (or outputs a measurement signal that can be processed to determine a heart rate of the subject over time). In some embodiments, the heart rate sensor 4 is a photoplethysmogram (PPG) sensor that comprises a light source for illuminating a part of the body of the subject (e.g. a finger, wrist, ear, forehead, etc., and a light sensor for measuring the light reflected and/or absorbed by the skin/tissue near the light source. The PPG sensor measures characteristics of the blood flow beneath the skin, and characteristics representing the pulse or heart beat of the subject can be extracted from the PGG measurement signal. In other embodiments, the heart rate sensor 4 can be an electrocardiogram (ECG) sensor that comprises one or more electrodes that are placed on the body of the subject and that measures the electrical activity of the heart. In other embodiments, the heart rate sensor 4 can be an accelerometer or other type of movement sensor that is placed in contact with a part of the body of the subject (e.g. chest, neck, wrist, finger, etc.) and that measures the accelerations (or more generally the movements) of the part of the body. An accelerometer (or other type of movement sensor) can be sensitive enough to movements of the skin caused by the pulsing of blood through blood vessels beneath the skin that the heart rate of the subject can be extracted from the measurement signal. In the case of an accelerometer, the accelerometer can measure the accelerations along three orthogonal axes (e.g. labelled X, Y and Z) and output three signals, each representing the accelerations along a respective one of the axes, or output a single signal that is a composite of the accelerations measured along the three orthogonal axes. The accelerometer 4 (or more generally the heart rate sensor 4) can operate with any suitable sampling frequency, for example 50 hertz (Hz), i.e. the accelerometer 4 can output an acceleration measurement every 1/50^{th} of a second, or for example 10 Hz. The accelerometer measurement signal can be processed using signal analysis techniques known in the art to extract movements due to a heart beat/pulsing of blood, and thus the heart rate of the subject can be derived from the acceleration measurements. Other types of movement sensor from which a heart rate measurement signal can be obtained include a gyroscope that measures changes in rotation and orientation. Those skilled in the art will be aware of other types of movement sensor that can be used to measure heart rate in an apparatus 2 according to the invention. Those skilled in the art will also be aware of other types of heart rate sensor that can be used to measure heart rate in an apparatus 2 according to the invention.

The measurements of (or signals representing) the heart rate are provided to a processing unit 6 in the apparatus 2. The processing unit 6 processes the measurements/signals to identify a urination event of the subject, as described in more detail below. The processing unit 6 also controls the operation of the apparatus 2, for example controlling the initiation of the collection of measurements by the heart rate sensor 4 and/or controlling other functions and operations of the apparatus 2. The processing unit 6 can be implemented in numerous ways, with software and/or hardware, to perform the various functions required. The processing unit 6 may comprise one or more microprocessors that may be programmed using software to perform the required functions. The processing unit 6 may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).

In various implementations, the processing unit 6 may be associated with one or more storage media, shown as memory unit 6 in Fig. 3. The memory unit 8 can be part of the processing unit 6, or it can be a separate component in the apparatus 2 that is connected to the processing unit 6. The memory unit 8 can comprise any suitable or desired type of volatile or non-volatile computer memory such as RAM, PROM, EPROM, and EEPROM. The memory unit 8 can be used for storing program code that can be executed by the processing unit 6 to perform the method described herein. The memory unit 8 can also be used to store signals or measurements from the heart rate sensor 4, and/or information relating to urination events identified by the processing unit 6.

As noted above, a urination event can be detected from measurements of the heart rate of the subject and measurements of the movements of the subject. In some embodiments, depending on the type of heart rate sensor 4 used in the apparatus 2, the heart rate sensor 4 (or the measurement signal from the heart rate sensor 4) can also be used to provide measurements of the movements of the subject. For example, in embodiments where the heart rate sensor 4 is a PPG sensor, the PPG measurement signal can be analysed to identify artefacts in the signal due to motion (e.g. where the PPG sensor moves relative to the skin of the subject, perhaps due to an impact from footsteps, etc., and it is difficult or not possible to extract a PPG pulse wave from the PPG measurement signal), and thus the PPG sensor acts as both a heart rate and movement sensor. Alternatively, in embodiments where the heart rate sensor 4 is an accelerometer or other type of movement sensor, the accelerometer measurement signal can also be analysed to identify an amount or level of movement of the subject, in addition to analysing the measurement signal to determine the heart rate. Those skilled in the art will be aware of other types of sensor that could measure both heart rate and movement of a subject. In either case, only one sensor is required to measure both parameters of the subject.

In alternative embodiments, the apparatus 2 can comprise a movement sensor 10 in addition to the heart rate sensor 4. The movement sensor 10 measures the movements or other motion of the subject, or movements or other motion of the part of the body of the subject that the apparatus 2 is worn on or carried by (e.g. the arm of the subject in the case of a wrist-worn or arm-worn apparatus 2). The movement sensor 10 outputs a movement signal indicative of the movements of the subject over time to the processing unit 6. In some embodiments the movement sensor 10 is an accelerometer that measures accelerations in three dimensions, however in other embodiments the movement sensor 10 can be a gyroscope that measures changes in rotation and orientation or a camera that records images of the subject or the subject's surroundings. In some embodiments, the apparatus 2 can comprise multiple types of movement sensor 10 (e.g. an accelerometer and gyroscope). Those skilled in the art will be aware of other types of movement sensor that can be used in an apparatus 2 according to the invention.

It will be appreciated that the apparatus 2 can comprise a movement sensor 10 separate from the heart rate sensor 4, regardless of the type of heart rate sensor 4 used in the apparatus 2. For example, the apparatus 2 can comprise a PPG sensor 4 for measuring heart rate, and an accelerometer 10 for measuring the movements of the subject.

Also as noted above, in some embodiments a urination event can be detected by monitoring heart rate and skin temperature. Depending on the type of heart rate sensor 4 (or movement sensor 10, if present) in the apparatus 2, it may be possible determine the skin temperature from the measurement signal obtained by the heart rate sensor 4 or movement sensor 10. For example, in embodiments where the heart rate sensor 4 is a PPG sensor, characteristics of the pulse or signal amplitude in the PPG signal can be used as the surrogate skin temperature measurement, since the volume of blood flow through blood vessels can be affected by the temperature of the skin. Suitable characteristics include the signal amplitude, peak-to-peak amplitude, and beat-to-beat amplitude fluctuation. In these embodiments, no separate skin temperature sensor is required. In alternative embodiments, the apparatus 2 can further comprise a skin temperature sensor 12 that measures the temperature of a part of the surface of the skin of the subject. The temperature sensor 12 outputs a skin temperature signal indicative of the skin temperature of part of the body of the subject over time. In some embodiments, the skin temperature sensor 12 comprises a temperature-sensitive element that is placed in contact with an area of the skin of the subject and that provides measurements of the temperature of the surface of the skin. Those skilled in the art will be aware of various types of temperature sensor 12 that can be used in the apparatus 2. Alternatively, the temperature sensor 12 can be an imaging device that is sensitive to infrared light and that images a part of the skin of the subject, and the temperature of the skin of the subject can be determined from the infrared images. In another alternative, rather than measure the temperature of the skin directly (e.g. by using a temperature-sensitive element in contact with the skin), the temperature sensor 6 can measure another physiological characteristic of the subject that is indicative of the skin temperature of the subject, or indicative of changes in skin temperature. In this case, the physiological characteristic is used as a surrogate measure for skin temperature. For example, the temperature sensor 12 can be a PPG sensor (e.g. in embodiments where the heart rate sensor 4 is not a PPG sensor, or as an additional PPG sensor to the heart rate sensor 4) that measures characteristics of the blood flow beneath the skin, and characteristics of the pulse or signal amplitude can be used as the surrogate skin temperature measurement. As with the heart rate sensor 4, the temperature sensor 12 can operate with any suitable sampling frequency, for example 50 Hz or 10 Hz. In some embodiments, the temperature sensor 12 has a measurement resolution of 0.02 degree Celsius (°C).

In some embodiments, the apparatus 2, including the heart rate sensor 4 (and movement sensor 10 and/or skin temperature sensor 12, if present), can be in a form that can be worn on or near the wrist, arm, hand or finger(s) of the subject. For example the apparatus 2 can be in the form of a watch or wrist band or strap. In these embodiments, the sensor(s) 4, 10, 12 measure the heart rate/movement/skin temperature on or near the wrist, or on the arm, hand or finger(s) of the subject. Alternatively, the apparatus 2, including the sensor(s) 4, 10, 12 can be in a form that can be worn on the chest or back of the subject. For example the apparatus 2 can be in the form of a patch that can be adhered or otherwise fixed to the subject. In these embodiments, the sensor(s) 4, 10, 12 measure the heart rate/movement/skin temperature on the chest or back of the subject.

In some embodiments the heart rate sensor 4 (and movement sensor 10 and/or skin temperature sensor 12) are part of the same device or housing as the processing unit 6, but in other embodiments the heart rate sensor 4 (and other sensors 10, 12, if present) are in a separate device or housing to the processing unit 6. Where the heart rate sensor 4 (and other sensors 10, 12, if present) are provided in a separate device or housing to the processing unit 6, appropriate circuitry or components can be provided to enable the measurement signals to be sent from the sensor(s) 4, 10, 12 to the processing unit 6. For example where the sensor(s) 4, 10, 12 are worn on or near the wrist of the subject, the processing unit 6 can be part of a smartphone or other electronic device that the subject carries in their pocket or wears on their chest, in which case the measurements from the sensor(s) 4, 10, 12 can be sent wirelessly to the processing unit 6 in the smartphone or other device so that the urination events can be identified.

In some embodiments the processing unit 6 can receive the measurements/signals directly from the heart rate sensor 4, movement sensor 10 (if present) and skin temperature sensor 12 (if present) and the processing unit 6 can process these measurements in real-time or near real-time in order to identify urination events of the subject. In other embodiments (including embodiments where the sensor(s) 4, 10, 12 are separate from the processing unit 6), the measurements from the sensor 4, 10, 12 can be stored in memory unit 8 and the processing unit 6 can retrieve and analyse the previously-obtained sensor measurements from the memory unit 8 when urination events of the subject are to be identified.

As noted above, in some embodiments the processing unit 6 may be part of a smart phone or other general purpose computing device that can be connected to or otherwise receive a measurement signal from heart rate sensor 4, movement sensor 10 (if present) and skin temperature sensor 12, but in other embodiments the apparatus 2 can be an apparatus that is dedicated to the purpose of identifying urination events of a subject. In embodiments where the processing unit 6 is part of a smart phone or other general purpose computing device, the heart rate sensor 4 (and movement sensor 10) could be the accelerometer, gyroscope and/or other type of movement sensor typically found in such a smart phone. The skin temperature sensor 12 (if present) could also be integrated into the smart phone or computing device, or provided separate to the smart phone or computing device so that it can provide skin temperature signals/measurements to the smart phone/computing device for processing and analysis (for example via a wired or wireless connection).

It will be appreciated that Fig. 3 only shows the components required to illustrate this aspect of the invention, and in a practical implementation the apparatus 2 will comprise additional components to those shown. For example, the apparatus 2 may comprise a battery or other power supply for powering the apparatus 2, a communication module for enabling the information on identified urination events to be communicated to a base unit for the apparatus 2 or to a remote computer, and/or one or more user interface components that allow the subject or another user to interact and control the apparatus 2. As an example, the one or more user interface components could comprise a switch, a button or other control means for activating and deactivating the apparatus 2 and/or urination event identification process. The user interface components can also or alternatively comprise a display or other visual indicator for providing information to the subject and/or other user about the operation of the apparatus 2, including displaying information on identified urination events.

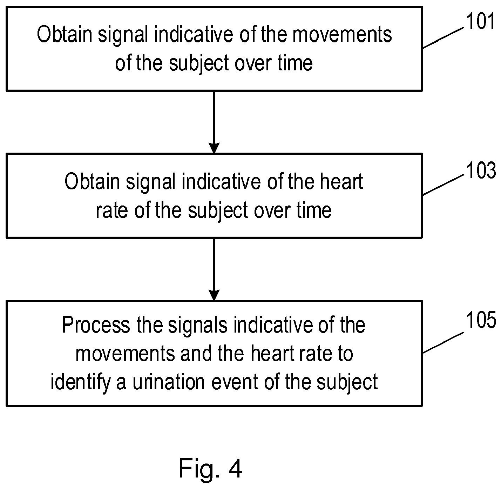

A method of monitoring a subject to identify urination events is shown in the flow chart of Fig. 4. The method can be performed by the apparatus 2, and particularly by the processing unit 6, for example in response to executing computer-readable code that is stored in the memory unit 8 or stored on some other suitable computer-readable medium, such as an optical disk, a solid state storage device, a magnetic-disk based storage device, etc.

In step 101 a signal indicative of the movements of the subject over time is obtained and in step 103 a signal indicative of the heart rate of the subject over time is obtained. It will be appreciated that the signal indicative of the movements and the signal indicative of the heart rate is obtained over corresponding (i.e. overlapping) time periods. Step 103 can comprise obtaining the heart rate signal directly using the heart rate sensor 4, or retrieving a signal made using the heart rate sensor 4 from the memory unit 8. Step 101 can comprise obtaining the signal indicative of the movements directly using the heart rate sensor 4 (in appropriate embodiments of the heart rate sensor 4) or a dedicated movement sensor 10 (if present), or retrieving a signal made using the heart rate sensor 4 or movement sensor 10 as appropriate from the memory unit 8. It will be appreciated that references in the following explanation of the invention to the measurements of the movements and measurements of the heart rate include reference to the movement signal and the heart rate signal respectively.

The signal indicative of the movements and the signal indicative of the heart rate are then processed to identify a urination event of the subject (step 105). Step 105 can be performed by processing unit 6. Generally, a urination event can be detected where there is a time period where a level of movement by the subject is below a threshold and the heart rate decreases.

An exemplary algorithm that can be used to identify urination events from movement signals and heart rate signals in step 105 is described below. It will be appreciated, however, that step 105 can be performed in other ways.

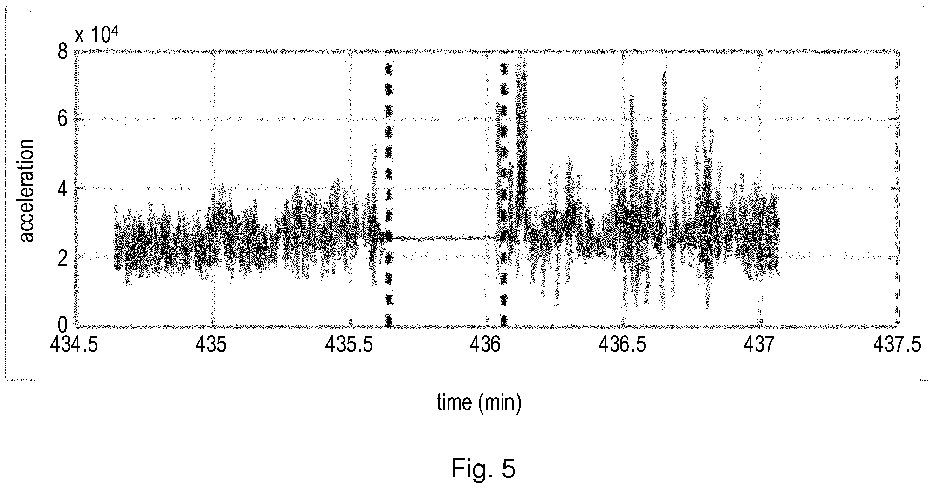

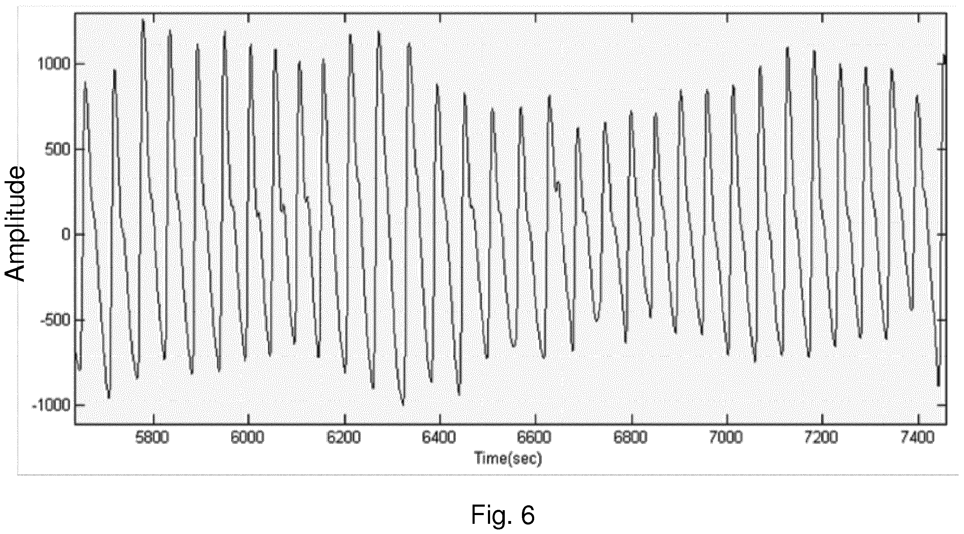

The graph in Fig. 5 illustrates a movement measurement signal from an accelerometer 10 (provided to measure the movements of the subject) that illustrates the accelerations before, during and after a urination event, and the graph in Fig. 6 illustrates an exemplary PPG signal that shows individual pulse waves (corresponding to individual heart beats). In the measurements of the movements of the subject in Fig. 5, the urination event occurred approximately between 435.6 minutes and 436.1 minutes, and it can be seen that there is little acceleration or activity by the subject during this time. It will be appreciated that a measurement signal exhibiting similar characteristics would be obtained from other types of movement sensors.

Since a urination event (i.e. the act of urinating) does not involve much movement by the subject (as shown in Fig. 5), the movement signal can be processed to identify time periods in which there is low activity/movement by the subject. Thus, in some embodiments the movement signal is processed to identify time periods in which the level of movement is below a threshold for the duration of the time period. The level of movement for a particular time instant can be given by the magnitude of the movement in the movement signal (e.g. the magnitude of the acceleration in the case of an acceleration signal), and a time period where there is low activity/movement can be identified where the magnitude of the movement is below the threshold for the duration of the time period. Alternatively the level of movement can be given by an activity count, movement count or energy that can be derived from the movement signal, and a time period where there is low activity/movement can be identified where the activity count, movement count or energy is below the threshold. It will be appreciated that an activity count, movement count or energy can be derived for a small amount of time (e.g. 1 second), and thus a time period where there is low activity/movement can be identified where the activity count, movement count or energy are below the threshold for the duration of the time period. Those skilled in the art will be aware of various techniques for determining a level of movement from movement signals, including from the magnitude, an activity count, movement count or energy.

In addition, a urination event is typically quite a short event (i.e. not lasting more than say, 1 minute in length), and typically having a duration from 15 to 60 seconds, with an average urination duration being around 28 seconds.

Therefore, in some embodiments, the movement signal can be processed to identify time periods of low activity/movement (i.e. where the level of movement is below the threshold) having a length that could correspond to a urination event. As such, in some embodiments, periods of low activity/movement that are much longer than a possible urination event (e.g. greater than 2 minutes) can be discarded at this stage of the algorithm. In other embodiments, only periods of low activity/movement that are within an acceptable range (e.g. between 5 and 60 seconds) are identified at this stage of the algorithm.

Furthermore, a urination event is normally preceded by the subject walking or otherwise moving to the toilet, and followed by the subject walking or otherwise moving away from the toilet. Thus, the periods of low activity/movement identified in this part of the algorithm should also be preceded and followed by periods of higher activity/movement (i.e. a level of movement/activity above the threshold) or detected walking (in embodiments where the movement signal is analysed to detect specific types of activities, such as walking, standing, sitting, etc.). These higher activity periods can also be seen in the signal in Fig. 5. It will be appreciated that the low level of movement/low activity and high level of movement/high activity may have respective thresholds, e.g. a low activity threshold below which the activity level (level of movement) is low, and a high activity threshold (that is higher than the low activity threshold) above which the activity level (level of movement) is high.

In some embodiments, the movement signal can be processed to detect or identify periods of time of a required duration (e.g. up to 2 minutes, between 5 and 60 seconds, etc.) in which there is low activity (low level of movement), and then the measurements either side of the detected period can be analysed to determine if they correspond to high activity or walking. Any low activity period (of the required duration) in the movement signal that is preceded and followed by high activity periods/walking periods can be identified as candidate urination events (i.e. the low activity period may correspond to a urination event).

In alternative embodiments, the movement signal can be analysed to detect high-low-high activity/movement/energy patterns using short time windows-based analysis. The window could, for example, be 3 minutes in length since that would cover the urination event and walking to the toilet prior to the event and walking away from the toilet after the event. Any window that shows a high activity → low activity → high activity pattern (where the high activity can be where the activity level (level of movement) is above a threshold and low activity can be where the activity level (level of movement) is below the or a respective threshold) may correspond to a urination event.

In window-based processing, the window is applied to the start of the movement signal (e.g. the first 3 minutes of measurements), and the measurements inside the window are analysed to determine if the required pattern described above is satisfied. The window can then be shifted along the movement measurement signal, for example shifted by 1 second, and the measurements inside the shifted window analysed, and so on.

In view of the information on the duration or urination events described above, any window of movement measurements that satisfies the high activity → low activity → high activity pattern, and where the low activity portion is of a required duration (e.g. no longer than 2 minutes, or between 5 and 60 seconds, etc.) can be considered to be a candidate urination event.

As noted above, it will be appreciated that where the movement measurements are acceleration measurements, low activity and high activity (low level of movement and high level of movement respectively) can correspond to the value or magnitude of the acceleration being below or above a threshold respectively. Alternatively, the acceleration measurements can be processed to determine an energy or activity level signal, and the energy or activity level compared to an appropriate threshold to identify the required high and low activity periods.

At this stage, it is not clear from the movement signal whether the candidate urination events actually correspond to urination events. For example, the low activity period between 435.6 minutes and 436.1 minutes in Fig. 5 could simply be a time period where the subject was standing still or sat down.

Therefore, the signal indicative of the heart rate can be used to determine if the candidate urination events identified using any of the techniques above correspond to an actual urination event. In some embodiments, the heart rate signal for time periods corresponding to the time periods of candidate urination events can be analysed to determine if the heart rate decreases, and if so, the candidate urination event can be classified as a urination event. In some embodiments, the heart rate signal for time periods corresponding to the time periods of candidate urination events can be analysed to determine if the heart rate decreases to the resting heart rate of the subject, below the resting heart rate of the subject or close to the resting heart rate of the subject, and if so, the candidate urination event can be classified as a urination event. This decrease of heart rate should occur shortly after urination onset, for example within the first 10 seconds of the start of urination. The decrease of heart rate should be temporary and the heart rate should return generally to the level prior to the urination event following the urination event. If the candidate urination event does not have a corresponding decrease in heart rate the candidate urination event can be discarded. In some embodiments, the decrease in the heart rate corresponding to a urination event is required to be a decrease to, or below, the resting heart rate of the subject. The decrease to or below the resting heart rate of the subject is not required to be instant (i.e. it is not required to drop instantaneously to or below the resting heart rate from the heart rate at the start of the candidate urination event), and the decrease can occur over a period of time, for example over a period of 10-30 seconds of the start of the candidate urination event. In these embodiments, it will be appreciated that information on the resting heart rate of the subject is required. In that case, the resting heart rate for the subject can be input to the apparatus 2 by the subject, by another user or received from another device or apparatus (e.g. that measures the resting heart rate of the subject or that has information on the resting heart rate stored therein), or the resting heart rate for the subject can be determined from a signal indicative of the heart rate obtained by the heart rate sensor 4 over a longer time period (e.g. one or more hours, one or more days, etc.). In some embodiments, as indicated above, the decrease in the heart rate corresponding to a urination event may be required to be a decrease in the heart rate across all or part of the urination period, rather than simply any drop in heart rate during the candidate urination period.

Thus, by making use of changes in movements of the subject and changes in heart rate of the subject, it is possible to detect urination events of the subject.

It will be appreciated that in alternative implementations of step 105, candidate urination events can be identified from the heart rate signal (e.g. periods up to a required duration (e.g. up to 2 minutes or between 5 and 60 seconds) where the heart rate decreases to or below the resting heart rate of the subject), and the movement measurements can be analysed to determine whether any of the candidate urination events are actual urination events (e.g. by determining if the decrease in heart rate to or below the resting heart rate coincides with a period of low activity, and optionally determining if the period of low activity is preceded or followed by periods of high activity).

Once one or more urination events are identified in the measurements, one or more characteristics of the urination event(s) can be determined. For example, each identified urination event can be analysed to determine the duration, where the duration is given by the duration of the heart rate decrease and/or the duration of the low activity period. As another example, the frequency of urination events can be determined once a number of urination events have been identified. The time of the urination event can be determined, and the time at which different urination events occurred compared. It is also possible to obtain information on whether the subject is having difficulty urinating by analysing the movement measurements in the low activity time period, for example analysing the signal energy to provide an indication of motion, which can be associated with difficulty or pain during the urination event. Information on stop-and-go patterns can be determined from the movement and heart rate signals. The characteristics of multiple urination events can be compared or evaluated to determine whether the characteristic is changing over time. Such changes can be indicative of improvements or worsening of a medical condition suffered by the subject.

In addition to the above processing and analysis, there are several optional steps that could be performed (either individually or in combination) to improve the accuracy of the urination event detection.

In a first optional step, a (candidate) urination event can only be detected where the subject is walking before and after the low activity period. As noted above, a urination event is typically preceded and followed by walking events. Although in the processing set out above it is necessary to determine if there are high activity periods before and after the low activity period corresponding to the urination event, in this optional step, the movement measurements are further analysed to determine if the subject is walking during those high activity periods. If it is determined that the subject is not walking during one or both of those high activity periods, then the candidate urination event can be discarded. Those skilled in the art will be aware of various techniques for detecting if the subject is walking from movement sensor measurements (particularly accelerometer measurements).

In a second optional step, a (candidate) urination event can only be detected where the subject is in a sitting or standing posture for the duration of the (candidate) urination event. Thus, in this optional step, the signal indicative of the movements is further analysed to determine if the subject is sitting or standing during the low activity period. If it is determined that the subject is not sitting or standing during the low activity period, then the candidate urination event can be discarded. Those skilled in the art will be aware of various techniques for detecting the posture of the subject from movement sensor measurements (particularly accelerometer measurements).

In a third optional step, further analysis of the signal indicative of the movements can be performed to detect other movements that are typical of visits to the toilet, such as opening and closing doors, washing hands and removing clothing. If the analysis of the signal indicative of the movements indicates that such movements have not occurred around the candidate urination event after the first walking/high activity period and before the second walking/high activity period, then the candidate urination event can be discarded. Those skilled in the art will be aware of various techniques for detecting particular movements or types of movements from movement sensor measurements (particularly accelerometer measurements).

In this third optional step, it will be appreciated that the analysis is effectively aiming to identify a portion of the movement signal having five distinctive parts, the low activity and walking periods described above (which each exhibit a generally regular movement pattern), and two high activity parts between the first walking period and the low activity period and the low activity period and the second walking period respectively that correspond to the other movements that are typical of visits to the toilet (and which are generally more irregular than activities such as walking).

As noted above, in addition to using heart rate signal and movement signal to detect urination events, in some embodiments measurements of skin temperature of a part of the body of the subject (e.g. the wrist, arm, hand or finger(s) of the subject) are obtained, and these skin temperature measurements (in the form of a signal indicative of the skin temperature) are analysed to detect a urination event. Therefore, in some embodiments, the signal indicative of the skin temperature can be used to determine if the candidate urination events identified above correspond to an actual urination event. In some embodiments, the skin temperature signal for time periods corresponding to the time periods of candidate urination events can be analysed to determine if the skin temperature decreases, and if so, the candidate urination event can be classified as a urination event. If the candidate urination event does not have a corresponding decrease in skin temperature and there is no corresponding decrease in the heart rate, the candidate urination event can be discarded. In some embodiments, the decrease in the skin temperature corresponding to a urination event may be required to be in a predetermined range, e.g. between 0.1°C and 1°C. In some embodiments, the decrease in the skin temperature corresponding to a urination event may be required to be a gradual decrease in the skin temperature across the whole urination period, rather than simply any drop in skin temperature during the candidate urination period. In some embodiments, a urination event can be detected where there is a decrease in the skin temperature during a candidate urination event, but no corresponding decrease in heart rate. Likewise, a urination event can be detected where there is a decrease in the heart rate during a candidate urination event, but no corresponding decrease in skin temperature.

In embodiments where a PPG sensor is used as either or both of the heart rate sensor 4 or movement sensor 10, a further feature can be extracted from the PPG measurement signal and used as a potential marker for urination events. In particular it has been found that the modulation depth of a PPG signal for a subject decreases during or at the start of a urination event. The modulation depth of a PPG signal is also referred to as the peak to peak amplitude of the PPG signal (i.e. the difference in amplitude between a maximum (peak) and the neighbouring minimum (a trough) in the PPG signal). Thus, a decrease in modulation depth means a decrease in the difference in amplitude between a maximum and the neighbouring minimum. The decrease in modulation depth of the PPG signal can be as a result of vasodilation (i.e. the widening of blood vessels), which is attributed to the urination event.

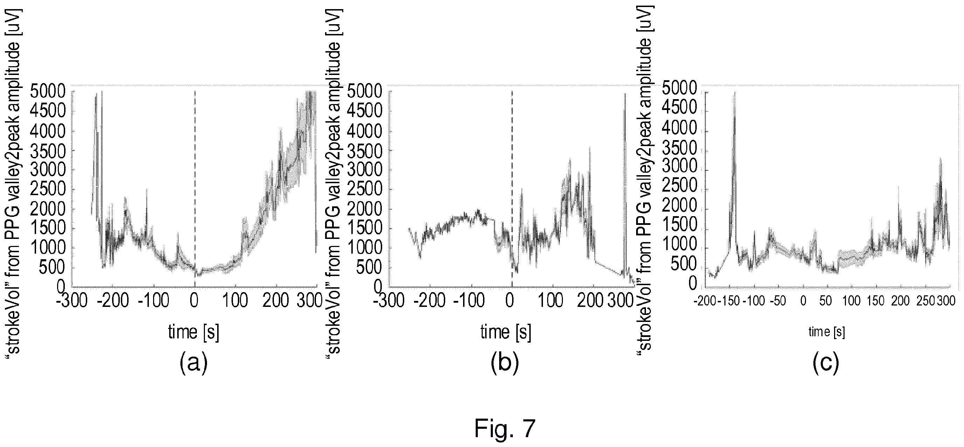

Fig. 7 comprises three graphs that each show a modulation depth signal derived from PPG measurements over a time period that included a urination event for the three different subjects mentioned above with reference to Figs. 1 and 2. As with Figs. 1 and 2, the time axes of each graph have been aligned so that the urination (micturition) event for each subject occurs at time = 0. It can be seen from each of the graphs that there is decrease in the modulation depth signal (and thus a decrease in the modulation depth) shortly before the urination event with the modulation depth being approximately at a minimum at the onset of the urination event (time = 0), with the modulation depth increasing following the urination event. Thus, in some embodiments, step 105 can further comprise analysing the PPG signal to determine the modulation depth over time, and a urination event can be detected at a time period where the modulation depth decreases and the movement signal indicates that the activity or movement of the subject is below a threshold. In some embodiments, the required decrease of the modulation depth can be of the order of 50% of the previous modulation depth base level. The decrease can also be required to happen within 30 seconds of the start of the urination event and be maintained for some time. In some embodiments, step 105 can be performed based on the signal indicative of the heart rate and the signal indicative of the movements as described above, and detecting a decrease of the modulation depth of the PPG signal can be used to improve the reliability of the detection of the urination event.

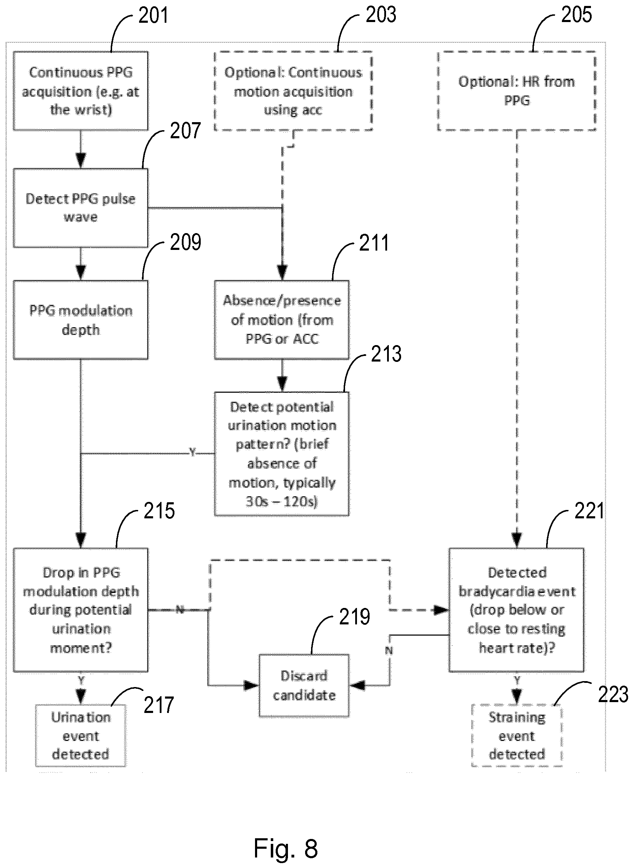

The flow chart in Fig. 8 illustrates another exemplary method of detecting urination events. This method can be performed by the apparatus 2, and particularly by the processing unit 6, for example in response to executing computer-readable code that is stored in the memory unit 8 or stored on some other suitable computer-readable medium, such as an optical disk, a solid state storage device, a magnetic-disk based storage device, etc. In this exemplary embodiment, a PPG sensor is used to obtain a PPG signal from which modulation depth is determined, and the heart rate of the subject is optionally be extracted from the PPG signal. An accelerometer 10 may be used to measure the movements of the subject.

In step 201, a PPG signal is acquired from the subject over time (e.g. continuously). The PPG sensor can be located at the wrist of the subject, although the PPG sensor could be positioned at other locations on the subject's body if required. Optionally, in step 203, an accelerometer 10 can be used to measure the movements of the subject over time (e.g. continuously). The accelerometer 10 can be positioned on the same part of the body of the subject as the PPG sensor (e.g. wrist), or it can be positioned on a different part. Also optionally, in step 205, the PPG signal can be analysed to extract or determine the heart rate of the subject (e.g. by determining the time between pulses in the PPG signal).

Next, in step 207, the PPG measurement signal is analysed to detect the PPG pulse wave, i.e. the part of the PPG signal corresponding to the heart beats of the subject. This results in a PPG signal as shown in Fig. 6. The PPG pulse wave is provided to step 209, and optionally also to step 211.

In step 209, the modulation depth of the PPG pulse wave is determined. The modulation depth is calculated or determined across the PPG pulse wave and results in a modulation depth signal similar to those shown in the graphs of Fig. 7.

In step 211 it is determined whether the subject is moving or at rest (or substantially at rest, i.e. the activity level is below a threshold). This can be determined from the PPG pulse wave signal detected in step 207, and/or it can be determined from a measurement signal from the accelerometer 10 obtained in step 203. In the case of determining whether the subject is moving or at rest from a PPG signal, step 211 can determine that the subject is in motion if it is not possible to detect a pulse wave in the PPG signal, and not in motion if it is possible to detect a pulse wave. Step 211 can comprise comparing a movement level to a threshold to determine whether the subject is in motion or at rest. Step 211 can be similar to step 105 described above.

In step 213 it is determined whether the movement signal/detected motion exhibits a pattern that is consistent with a urination event, e.g. a brief period where there is an absence of motion, e.g. with a duration of typically 30s to 120s. In other words, step 213 can aim to identify short period of time where there is an absence of movement (during micturition) "encapsulated" or surrounded by periods of movement (e.g. representing the subject going to and returning from the bathroom). If there is such a period, then the method can pass to step 215 where the PPG modulation depth signal determined in step 209 is analysed to determine if there is a drop (decrease) or decreasing trend in the modulation depth at a time corresponding to the identified period. If there is a corresponding drop in modulation depth, then the period can be identified as a urination event (step 217).

If there is no corresponding drop in modulation depth (or no drop in modulation depth of a required magnitude), then the method can pass to step 219 from step 215 in which the period is discounted or discarded as a possible urination event.

Alternatively, where the heart rate is extracted from the PPG signal in step 205, the method can pass from step 215 to step 221 in which it is determined whether there is an event in which there is a drop in heart rate to below or close to the resting heart rate of the subject at a time corresponding to the identified period where the motion is consistent with a urination event. If not, the method passes to step 219 where the period is discounted or discarded. If there is such an event then the period can be identified as a urination event (step 223). In particular embodiments, as such an event may be associated with the subject straining to urinate, detecting such an event in step 221 can lead to a urination event with straining being detected in step 223.

On detection of a urination event in step 217 or 223, the urination event can be analysed as described above, for example to determine information such as duration, trends in a series of urination events, etc.

There is therefore provided an improved method and apparatus for monitoring a subject to identify urination events of the subject.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of monitoring a subject, the method comprising:
obtaining, from a sensor, a signal indicative of the movements of the subject over time;
obtaining, from a sensor, a signal indicative of the heart rate of the subject over time; and
processing the obtained signal indicative of the movements and the obtained signal indicative the heart rate to identify a urination event of the subject as a time period where a level of movement by the subject is below a threshold and the heart rate decreases.

2. A method as defined in claim 1, wherein the step of processing comprises identifying a urination event of the subject as a time period where a level of movement by the subject is below a threshold and the heart rate decreases to close to a resting heart rate of the subject, to the resting heart rate of the subject, or below the resting heart rate of the subject.

3. A method as defined in claim 2, wherein the method further comprises one of:
(i) processing the signal indicative of the heart rate of the subject over time to determine the resting heart rate of the subject; and
(ii) receiving a measurement of the resting heart rate of the subject.

4. A method as claimed in claim 1, 2 or 3, wherein the step of processing the obtained signal indicative of the movements further comprises processing the obtained signal indicative of the movements to detect periods of time in which the subject is walking; and identifying the urination event as a time period where the level of movement by the subject is below a threshold and the heart rate decreases, and where the time period is preceded and followed by a period of time in which the subject is detected to be walking.

5. A method as claimed in claim 1, 2 or 3, wherein the step of processing the obtained signal indicative of the movements further comprises processing the obtained signal indicative of the movements to detect the posture of the subject over time; and identifying the urination event as a time period where the level of movement by the subject is below a threshold and the heart rate decreases, and where the subject is in a sitting or standing posture.

6. A method as claimed in any preceding claim, wherein the step of processing further comprises:
processing a PPG measurement signal from a PPG sensor to determine a signal representing the modulation depth of the PPG measurement signal over time; and
identifying a urination event of the subject as a time period where the level of movement by the subject is below the threshold and (i) the heart rate decreases; and/or (ii) the modulation depth decreases.

7. A method as claimed in any preceding claim, wherein the method further comprises the step of:
obtaining, from a sensor, a signal indicative of the skin temperature of the subject over time;
wherein the step of processing comprises processing the obtained signal indicative of the skin temperature to identify time periods in which the skin temperature decreases; and identifying a urination event of the subject as a time period where a level of movement by the subject is below the threshold and (i) the heart rate decreases; and/or (ii) the skin temperature decreases.

8. A method as claimed in any preceding claim, wherein the method further comprises the step of analysing one or more identified urination events to determine one or more characteristics of the urination event or events, the one or more characteristics comprising one or more of the duration of the urination event; the time of the urination event; the frequency with which urination events occur; an indication of pain, discomfort, stress, anxiety, emotional fluctuations and/or difficulty for the subject before, during and/or after the urination event.

9. A computer program product comprising a computer readable medium having computer readable code embodied therein, the computer readable code being configured such that, on execution by a suitable computer or processor, the computer or processor is caused to perform the method of any of claims 1-8.

10. An apparatus for monitoring a subject, the apparatus comprising a processing unit configured to:
obtain a signal indicative of the movements of the subject over time;
obtain a signal indicative of the heart rate of the subject over time; and
process the obtained signal indicative of the movements and the obtained signal indicative of the heart rate to identify a urination event of the subject as a time period where a level of movement by the subject is below a threshold and the heart rate decreases.

11. An apparatus as defined in claim 10, wherein the processing unit is configured to identify a urination event of the subject as a time period where a level of movement by the subject is below a threshold and the heart rate decreases to close to a resting heart rate of the subject, to the resting heart rate of the subject, or below the resting heart rate of the subject.

12. An apparatus as defined in claim 11, wherein the processing unit is further configured to:
(i) process the signal indicative of the heart rate of the subject over time to determine the resting heart rate of the subject; or
(ii) obtain a measurement of the resting heart rate of the subject.

13. An apparatus as claimed in claim 10-12, wherein the processing unit is configured to process the obtained measurements of movements to detect periods of time in which the subject is walking; and identify the urination event as a time period where the level of movement by the subject is below a threshold and the heart rate decreases, and where the time period is preceded and followed by a period of time in which the subject is detected to be walking.

14. An apparatus as claimed in any of claims 10-13, wherein the processing unit is configured to:
obtain the signal indicative of the movements of the subject from a photoplethysmogram, PPG, sensor, or a movement sensor; and
obtain the signal indicative of the heart rate of the subject from the PPG sensor, an electrocardiogram, ECG, sensor or the movement sensor.

15. A system for monitoring a subject, the system comprising:
an apparatus according to any of claims 10-14; and
one or more sensors for providing the signal indicative of the heart rate of the subject and the movements of the subject.
